# EUROPÄISCHE PATENTANMELDUNG

(11) **EP 2 319 482 A2**
(43) Veröffentlichungstag der Anmeldung: **11.05.2011**
(21) Anmeldenummer: 10178392.6
(22) Anmeldetag: 22.09.2010
(51) Int. Cl.: A61K 8/06, A61K 8/89, A61Q 1/12

(54) **Kosmetische W/S-Emulsion**

(30) Priorität: 28.10.2009 DE 102009051054
(71) Anmelder: Beiersdorf AG, 20253 Hamburg (DE)
(72) Erfinder: Münchow, Lynn, 22547, Hamburg (DE); Lemmer, Katharina, 20259, Hamburg (DE); Kruse, Uta, 22609, Hamburg (DE)

(57) **Zusammenfassung**

Kosmetische Wasser-in-Silikonöl-Emulsion (W/S-Emulsion) enthaltend
a) einen PEG/PPG-Dimethicon-Emulgator, bei dem das Verhältnis von PEG/PPG 4/3 beträgt,
b) ein oder mehrere Pigmente.

## Beschreibung

Die vorliegende Erfindung betrifft eine kosmetische Wasser-in-Silikonöl Emulsion enthaltend einen PEG/PPG-Dimethicon-Emulgator, bei dem das Verhältnis von PEG/PPG 4/3 beträgt sowie ein oder mehrere Pigmente.

Der Wunsch, schön und attraktiv auszusehen, ist seit Tausenden von Jahren in den Menschen verwurzelt. Auch wenn das Schönheitsideal im Laufe der Zeit Wandlungen erfahren hat, so ist das Streben nach einem makellosen Äußeren, immer das Ziel der Menschen gewesen, da ein sympathisches Erscheinungsbild ihr Selbstwertgefühl und die Anziehungskraft auf ihre Mitmenschen erhöht.

Der Begriff der dekorativen Kosmetik leitet vom lateinischen "decoratio" - das Hervorheben des Schönen - ab. Meist werden dabei mit Hilfe von Farbstoffen einzelne Körperpartien, insbesondere im Gesicht, hervorgehoben und farbliche Uneinheitlichkeiten abgemildert.

Das Gesichts-Make-up soll der Gesichtshaut ein natürliches Aussehen verleihen, blasse Haut auffrischen und farbliche Unregelmäßigkeiten der Haut ausgleichen.

Neben Gesichtspudern und Rouge als pulverförmigen Kosmetika, werden hierzu cremeförmige Präparate, wie Tagescremes und Creme-Make-up auf Emulsionsbasis verwendet. Eine besondere Ausführungsform ist die sogenannte Foundation. Als Foundation bezeichnet man dabei flüssige oder halbfeste Make-up-Präparate, die meist hautfarben sind (d.h. die Haut tönen) und auf das Gesicht, insbesondere auf die Wangen aufgetragen werden. Sie verleihen diesen ein gleichmäßiges gesundes gebräuntes oder rötliches Aussehen. Ihren Farbton erhalten diese Zubereitungen in der Regel durch eine Mischung aus Farbpigmenten (in der Regel Kombinationen mit Eisenoxid). Häufig werden Foundations auf der Basis von Wasser-in-Silikonöl-Emulsionen (W/S-Emulsionen) formuliert.

Nachteilig am Stande der Technik ist der Umstand, dass herkömmliche Foundations auf W/S-Emulsionsbasis bei höherem Pigmentgehalt (größer/gleich 10 Gew.-%) eine sehr hohe Viskosität aufweisen und sich nur noch schwer gleichmäßig auf der Haut verteilen lassen. Die Einsatzkonzentration ist bei kommerziellen Produkten damit praktisch auf Pigmentgehalte unter 10 Gew.-% beschränkt, da die Verbraucherinnen hochviskose und schwer verteilbare Foundations nicht akzeptieren. Dies führt dazu, dass herkömmliche Foundations nicht ausreichend pigmentiert werden können und die Verbraucherinnen in relativ großen Mengen und in dicken Schichten auftragen müssen. Da die Verbraucherinnen von einer handelsüblichen Foundation darüber hinaus eine gewisse Mindestanwendbarkeitshäufigkeit erwarten, müssen die herkömmlichen Foundations in relativ großen und wenig "Handtaschen-tauglichen" Verpackungsbehältnissen angeboten werden.

Es war daher die Aufgabe der vorliegenden Erfindung, die Mängel des Standes der Technik zu beseitigen und eine Foundation zu entwickeln, die trotz hoher Pigmentgehalte eine ausreichend dünnflüssige Viskosität und leichte Verteilbarkeit auf der Haut gewährt. Die Zubereitung sollte darüber hinaus besonders angenehm auf der Haut zu tragen sein.

Überraschend gelöst werden die Aufgaben durch eine kosmetische Wasser-in-Silikonöl-Emulsion (W/S-Emulsion) enthaltend
a) einen PEG/PPG-Dimethicon-Emulgator, bei dem das Verhältnis von PEG/PPG 4/3 beträgt,
b) ein oder mehrere Pigmente, sowie
durch die Verwendung von PEG/PPG-Dimethicon-Emulgatoren, bei denen das Verhältnis von PEG/PPG 4/3 beträgt, zur Verbesserung der Verteilbarkeit pigmenthaltiger kosmetischer Wasser-in-Silikonöl-Emulsionen (W/S-Emulsionen) auf der Haut.

Die erfindungsgemäßen Zubereitungen lassen sich überraschend einfach und kostengünstig herstellen.

Die in dieser Beschreibung der Erfindung als mit "erfindungsgemäß", "erfindungsgemäß vorteilhaft" etc. gekennzeichneten Angaben beziehen sich sowohl auf die erfindungsgemäße Zubereitung als auch auf die erfindungsgemäße Verwendung.

Als Wasser-in-Silikonöl Emulsion werden erfindungsgemäß Wasser-in-Öl-Emulsionen verstanden, deren Silikonöl-Anteil in der Ölphase mindestens 50 Gewichts-%, bezogen auf das Gesamtgewicht der Ölphase beträgt.

Es ist erfindungsgemäß bevorzugt, wenn als PEG/PPG-Dimethicon-Emulgator, bei dem das Verhältnis von PEG/PPG 4/3 beträgt, PEG/PPG 20/15 Dimethicon eingesetzt wird.

Dieses Dimethicon-Derivat, welches die INCI Cyclopentasiloxane (and) PEG/PPG-20/15 Dimethicone trägt, kann beispielsweise bei der Firma Momentive performance materials unter dem Kürzel SF1540 erhalten werden.

Es ist erfindungsgemäß vorteilhaft, wenn die erfindungsgemäße Zubereitung mindestens 10 Gew.-%, bezogen auf das Gesamtgewicht der Zubereitung, an Pigmenten enthält. Es ist erfindungsgemäß bevorzugt, wenn die Zubereitung mindestens 15 Gew.-%, bezogen auf das Gesamtgewicht der Zubereitung, an Pigmenten enthält.

Grundsätzlich kann die erfindungsgemäße Zubereitung die in der Kosmetik üblichen Pigmente enthalten.

Es ist erfindungsgemäß vorteilhaft, wenn als Pigmente Metalloxide eingesetzt werden.

Eine erfindungsgemäß besonders vorteilhafte Ausführungsform der vorliegenden Erfindung ist dadurch gekennzeichnet, dass die Zubereitung ein schichtförmig ummantelt aufgebautes Mischpigment aus Mica, Bismuthoxychlorid und Eisenoxid enthält.

Dieses ist erfindungsgemäß bevorzugt dadurch gekennzeichnet, dass das Mischpigment einen Kern aus Mica enthält, der mit Bismuthoxychlorid ummantelt ist, welches wiederum außen mit Eisenoxid beschichtet ist.

Es ist erfindungsgemäß bevorzugt, wenn die Pigmentphase der Zubereitung aus einer Mischung aus Titandioxid und einem schichtförmig aufgebauten Mischpigment aus Mica, Bismuthoxychlorid und Eisenoxid besteht, wobei, besonders bevorzugt, das Mischpigment einen Kern aus Mica enthält, der mit Bismuthoxychlorid ummantelt ist, welches wiederum außen mit Eisenoxid beschichtet ist.

Es ist erfindungsgemäß vorteilhaft, wenn die Zubereitung einen PEG/PPG-Dimethicon-Emulgator, bei dem das Verhältnis von PEG/PPG 4/3 beträgt, in einer Konzentration von 0,5 bis 2,5 Gew.-%, bezogen auf das Gesamtgewicht der Zubereitung, enthält.

Es ist erfindungsgemäß bevorzugt, wenn die Zubereitung einen PEG/PPG-Dimethicon-Emulgator, bei dem das Verhältnis von PEG/PPG 4/3 beträgt, in einer Konzentration von 1 bis 2 Gew.-%, bezogen auf das Gesamtgewicht der Zubereitung, enthält.

Die Konzentrationsangaben beziehen sich dabei auf den Aktivgehalt des Emulgators.

Es ist erfindungsgemäß vorteilhaft, wenn die Zubereitung das schichtförmig ummantelt aufgebaute Mischpigment aus Mica, Bismuthoxychlorid und Eisenoxid, in einer Konzentration von 1 bis 6 Gew-% bezogen auf das Gesamtgewicht der Zubereitung, enthält.

Enthält die Zubereitung erfindungsgemäß vorteilhaft mindestens 10 Gewichts-% (bzw. mindestens 15 Gewichts-%) an Pigmenten, sind also neben diesem Mischpiment weitere Pigmente in der Zubereitung enthalten.

Erfindungsgemäß vorteilhafte Ausführungsformen sind auch dadurch gekennzeichnet, dass die Zubereitung als weitere Bestandteile einen oder mehrere der Verbindungen gewählt aus den Verbindungen Silica, Lauryllysin, Squalan, Propylencarbonat enthält.

Ferner ist es erfindungsgemäß vorteilhaft, wenn die Zubereitung einen oder mehrere Füllstoffe enthält. Erfindungsgemäß bevorzugt werden die Füllstoffe gewählt aus der Gruppe der Verbindungen Disteardimoniumhectorid, Nylon 6/12, Silica, Aluminiumstärkeoctenylsuccinate, Dimethicon-Crosspolymer, Dimethicon/Vinyldimethicon-Crosspolymer, Methylmethacrylat-Crosspolymer, Talkum, Reisstärke und/oder Polymethylsilsesquioxane.

Erfindungsgemäß vorteilhaft ist es, wenn die Zubereitung ein oder mehrere Antioxidantien gewählt aus der Gruppe der Verbindungen Tocopherolacetat, Tocopherol, 2,6-Di-*tert*-butyl-4-methylphenol enthält.

Nicht zuletzt enthält die erfindungsgemäße Zubereitung vorteilhaft ein oder mehrere Parfümstoffe, UV-Filter und/oder Konservierungsmittel (beispielsweise Methylparaben, Ethylparaben, Propylparaben, Butylparaben, Phenoxyethanol, Isobutylparaben, Diazolidinylharnstoff).

Die Wasserphase der erfindungsgemäßen Zubereitungen kann vorteilhaft übliche kosmetische Hilfsstoffe enthalten, wie beispielsweise Alkohole, insbesondere solche niedriger C-Zahl, vorzugsweise Ethanol und/oder Isopropanol, Diole oder Polyole niedriger C-Zahl sowie deren Ether, vorzugsweise Propylenglykol, 2-Methylpropan-1,3-diol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, Polymere, Schaumstabilisatoren, Elektrolyte sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z. B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination. Weitere erfindungsgemäß vorteilhafte Verdicker sind Permulen TR 1, TR 2, Carbopol 1328, Aristoflex AVC.

Die erfindungsgemäße Ölphase enthält erfindungsgemäß Dimethicon und/oder Cyclomethicon. Sie kann darüber hinaus auch noch weitere Öl- und/oder Wachskomponenten enthalten, beispielsweise Myristyllactat, Dicaprylylcarbonat, Octyldodecanol, Trimethylsiloxysilikat, Sonnenblumenöl, Avocadoöl, Caprin / Capric Triglycerid, Kohlenwasserstoffe wie Isododecan, Isohexadecan, Polyisobuten, Paraffine.

Erfindungsgemäß vorteilhaft enthält die erfindungsgemäße Zubereitung einen oder mehrere Füll- und/oder Puderstoffe. Diese werden erfindungsgemäß bevorzugt gewählt aus der Gruppe der Verbindungen Talkum, Nylon, Silica, Methyl Methacrylate Crosspolymer, Reisstärke und/oder Polymethylsilsesquioxane.

### Vergleichsversuche

Die folgenden Vergleichsversuche können die erfindungsgemäßen Effekte verdeutlichen, wobei Beispiel I erfindungsgemäß ist, Beispiele II und III als Vergleich aufgeführt werden:

### Flüssige pigmentierte Grundierung

| | | I | II | III |
|---|---|---|---|---|
| A | Wasser | Add 100 | Add 100 | Add 100 |
| | Glycerin | 5 | 5 | 5 |
| | Natriumchlorid | 2 | 2 | 2 |
| | | | | |
| B | Cyclomethicon | 23,4 | 23,4 | 23,4 |
| | Dicaprylyl Carbonat | 6 | 6 | 6 |
| | PEG/PPG 20/15 Dimethicon (aktiv) SF 1540¹ | 1,6 | | |
| | Cetyl PEG/PPG-10/1 Dimethicon Abil EM 90² | | 1,6 | |
| | PEG/PPG-19/19 Dimethicon (aktiv) BY11-030³ | | | 1,6 |
| | Disteardimonium Hectorit | 0,5 | 0,5 | 0,5 |
| | Propylen Carbonat | 0,1 | 0,1 | 0,1 |
| | | | | |
| C | CI 77891 (Titandioxid) | 9 | 9 | 9 |
| | CI 77492 + Cl 77491 + Cl 77499 | 1 | 1 | 1 |
| | Mica + Cl 77163 + Cl 77491 + Cl 77492 + Cl 77499 (Mischpigment)⁴ | 5 | 5 | 5 |
| | Parfum | 0,3 | 0,3 | 0,3 |
| | Silica | 3 | 3 | 3 |
| | | | | |
| | Viskosität (25°C) | 2700 mPas | 1300 mPas | 2700 mPas |

| | | | | |
|---|---|---|---|---|
| ¹Momentive ²Evonik Goldschmidt ³Dow Corning ⁴Mischpigment mit einem Kern aus Mica, der mit Bismuthoxychlorid ummantelt ist, welches wiederum außen mit Eisenoxid beschichtet ist | | | | |

Herstellung: Phase C wird in Phase B gründlich dispergiert. Unter Energieeintrag wird Phase A zur Mischung B+C zugegeben. Es entsteht eine homogene Wasser-in-Silikonöl Emulsion.

Die Viskositäten werden 1 Tag nach Herstellung der Emulsion mit einem Rotationsviskosimeter gemessen.

### Ergebnis:

Die Emulsion aus Bespiel I lässt sich sehr leicht und angenehm auf der Haut verteilen. Die Pigmente hinterlassen einen gleichmäßigen Farbeindruck.

Die Emulsion aus Bespiel II zeigt bereits nach 4 Wochen Lagerung bei 25°C eine Phasentrennung.

Die Emulsion aus Bespiel III erweist sich als zäh beim Verteilen auf der Haut. Die Pigmente hinterlassen einen streifigen Eindruck, nicht gleichmäßigen Eindruck.

### Beispiele

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

### Flüssige pigmentierte Grundierung

| | | IV | V | VI | VII | VIII |
|---|---|---|---|---|---|---|
| A | Wasser | Add 100 | Add 100 | Add 100 | Add 100 | Add 100 |
| | Glycerin | 5 | 6 | 5 | 8 | 5 |
| | Natriumchlorid | 2 | | 1 | 2 | 1 |
| | | | | | | |
| B | Cyclomethicon | 23 | 22 | 17 | 24 | 23 |
| | Dimethicon 100 cst | | | 2 | | |
| | Squalan | | 0,5 | | | |
| | Dicaprylyl Carbonat | 6 | 3 | | 5 | 6 |
| | Dicaprylyl Ether | | 3 | 6 | 1 | |
| | Shea Butter | | | | | 0,5 |
| | Octyl Methoxy Cinnamat | | 2 | | | |
| | PEG/PPG 20/15 Dimethicon (aktiv) SF 1540 | 1,6 | 2 | 1,5 | 1,8 | 2,5 |
| | Disteardimonium Hectorit | 0,5 | 0,5 | | 0,5 | |
| | Propylen Carbonat | 0,1 | 0,1 | | 0,1 | |
| | | | | | | |
| C | Cl 77891 (Titandioxid) | 9 | 10 | 7 | 8 | 5 |
| | Cl 77492 + Cl 77491 + Cl 77499 | 1 | 1,2 | 2 | 2 | 1 |
| | Mica + Cl 77163 + CI 77491 + CI 77492 + Cl 77499 (Mischpigment)⁴ | 4 | 3 | 3 | | 5 |
| | Mica + Cl 77163 + Cl 77491 (Mischpigment)⁴ | 1 | | | | 2 |
| | Mica + Cl 77163 + Cl 77492 (Mischpigment)⁴ | | | 3 | | 2 |
| | Parfum | 0,3 | 0,2 | | 0,1 | 0,2 |
| | Konsvervierung | q.s. | q.s. | q.s. | q.s. | q.s. |
| | Silica | 3 | 1 | 2 | 4 | |
| | Lauroyl Lysine | | 1 | | | 2 |
| | Methyl Methacrylate Crosspolymer | | | 1 | | 2 |

| | | | | | | |
|---|---|---|---|---|---|---|
| ⁴ Mischpigment mit einem Kern aus Mica, der mit Bismuthoxychlorid ummantelt ist, welches wiederum außen mit Eisenoxid beschichtet ist | | | | | | |

Herstellung: Phase C wird in Phase B gründlich dispergiert. Unter Energieeintrag wird Phase A zur Mischung B+C zugegeben. Es entsteht eine homogene Wasser-in-Silikonöl Emulsion.

Die Emulsionen zeichnen sich durch eine äußerst gute Verteilbarkeit und sehr gleichmäßige Deckkraft aus. Das Hautgefühl ist zunächst angenehm ölig, später puderig und angenehm.

## Patentansprüche

1. Kosmetische Wasser-in-Silikonöl-Emulsion (W/S-Emulsion) enthaltend
a) einen PEG/PPG-Dimethicon-Emulgator, bei dem das Verhältnis von PEG/PPG 4/3 beträgt,
b) ein oder mehrere Pigmente.

2. Verwendung von PEG/PPG-Dimethicon-Emulgatoren, bei denen das Verhältnis von PEG/PPG 4/3 beträgt, zur Verbesserung der Verteilbarkeit pigmenthaltiger kosmetischer Wasser-in-Silikonöl-Emulsionen (W/S-Emulsionen) auf der Haut.

3. Kosmetische Zubereitung nach Anspruch 1 oder Verwendung nach Anspruch 2, **dadurch gekennzeichnet, dass** als PEG/PPG-Dimethicon-Emulgator PEG/PPG 20/15 Dimethicon eingesetzt wird.

4. Kosmetische Zubereitung oder Verwendung nach einem der vorhergehenden Ansprüche, **dadurch gekennzeichnet, dass** die Zubereitung mindestens 10 Gew.-%, bezogen auf das Gesamtgewicht der Zubereitung, an Pigmenten enthält.

5. Kosmetische Zubereitung oder Verwendung nach einem der vorhergehenden Ansprüche, **dadurch gekennzeichnet, dass** die Zubereitung mindestens 15 Gew.-%, bezogen auf das Gesamtgewicht der Zubereitung, an Pigmenten enthält.

6. Kosmetische Zubereitung oder Verwendung nach einem der vorhergehenden Ansprüche, **dadurch gekennzeichnet, dass** als Pigmente Metalloxide eingesetzt werden.

7. Kosmetische Zubereitung oder Verwendung nach einem der vorhergehenden Ansprüche, **dadurch gekennzeichnet, dass** die Zubereitung ein schichtförmig ummantelt aufgebautes Mischpigment aus Mica, Bismuthoxychlorid und Eisenoxid enthalten.

8. Kosmetische Zubereitung oder Verwendung nach Anspruch 7, **dadurch gekennzeichnet, dass** das Mischpigment einen Kern aus Mica enthält der mit Bismuthoxychlorid ummantelt ist, welches wiederum außen mit Eisenoxid beschichtet ist.

9. Kosmetische Zubereitung oder Verwendung nach einem der vorhergehenden Ansprüche, **dadurch gekennzeichnet, dass** die Pigmentphase der Zubereitung eine Mischung aus Titandioxid und einem schichtförmig aufgebauten Mischpigment aus Mica, Bismuthoxychlorid und Eisenoxid besteht.

10. Kosmetische Zubereitung oder Verwendung nach einem der vorhergehenden Ansprüche, **dadurch gekennzeichnet, dass** die Zubereitung einen PEG/PPG-Dimethicon-Emulgator, bei dem das Verhältnis von PEG/PPG 4/3 beträgt, in einer Konzentration von 0,5 bis 2,5 Gew.-%, bezogen auf das Gesamtgewicht der Zubereitung, enthält.

11. Kosmetische Zubereitung oder Verwendung nach einem der vorhergehenden Ansprüche, **dadurch gekennzeichnet, dass** die Zubereitung als weitere Bestandteile einen oder mehrere der Verbindungen gewählt aus den Verbindungen Lauryllysin, Squalan, Propylencarbonat, Sheabutter enthält.
